# EUROPEAN PATENT APPLICATION

(11) **EP 1 938 835 A1**
(43) Date of publication of application: **02.07.2008**
(21) Application number: 06027120.2
(22) Date of filing: 29.12.2006
(51) Int. Cl.: A61K 39/145

(54) **Non-specific immunostimulating agents**

(71) Applicant: Pevion Biotech AG, 3018 Bern (CH)
(72) Inventor: Moser, Christian, Dr., 3122 Kehrsatz (CH); Kammer, Andreas, Dr., 3052 Zollikofen (CH); Zurbriggen, Rinaldo, Dr., 3185 Schmitten (CH)
(74) Representative: Grünecker, Kinkeldey, Stockmair & Schwanhäusser Anwaltssozietät

(57) **Abstract**

The present invention relates to a lipid vesicle. The invention further relates to the use of such a lipid vesicle for the preparation of a medicament. The invention further relates to a method of treating and/or preventing a disease or disorder involving administering such a lipid vesicle to an animal in need thereof.

## Description

The present invention relates to a lipid vesicle. The invention further relates to the use of such a lipid vesicle for the preparation of a medicament. The invention further relates to a method of treating and/or preventing a disease or disorder involving administering such a lipid vesicle to an animal in need thereof.

It is desirable to increase the general resistance against diseases, especially against infectious diseases by means of non-specific stimulation of the body's immune system. At the same time the importance of the immune system for the suppression and elimination of neoplastic diseases has become evident and generally accepted. However, the mechanisms of action of general immunostimulants are unknown and subject to speculation, due to the often complex compositions and the multiple interactions with different organ systems. The increasing understanding of the interplay between innate defense mechanisms and adaptive immunity has provided plausible explanations. In particular the discovery of toll-like receptors in the 1990s, a sensor system for danger signals, has initiated a new field of research dealing with highly complex, integrated and multi-redundant defense mechanisms against invading microorganisms that provides plausible - yet incomplete - explanations for enhanced resistance against disease.

Many potential immune enhancers have been described in the literature, ranging from small synthetic molecules (poly I:C, Levamisole) to living microorganisms (Corynebacterium parvum), and including complex mixtures of bacterial components with mineral oils (Freund's adjuvant) or inorganic salts (aluminum and magnesium hydroxide/phosphate) and, more recently, recombinant proteins modulating immunity (eg cytokines, antibodies against cellular receptors). These substances have been used predominantly as adjuvants for vaccines, i.e. in combination with a specific antigen for the purpose of enhancing the immunity/resistance against the disease with which the antigenic components of the vaccine are associated. Among the large number of known immunostimulants, only very few are actually accepted adjuvants for human vaccines (Alum salts, virosomes, MF59, RC529). Most approaches have not progressed beyond pure preclinical research, leaving relevant regulatory (safety, toxicity) and economic (production costs, product profiles) aspects unaddressed. Other adjuvants are in varying stages of clinical development (e.g. bacterial toxins, Saponin derivatives, LPS and Lipid A derivatives, CpGs, nanoparticles) or are accepted for veterinary use only (e.g.

ISCOMS, GERBU). The problem is to strike an acceptable balance between stimulatory effect and reactogenicity, thus the capacity to produce adverse reactions. Newly developed adjuvants bear the additional risk of unexpected side effects, as for example observed with heat labile toxin from E.coli as an adjuvant for a nasal influenza vaccine.

None of the adjuvants approved for specific vaccines is used to stimulate disease resistance in a non-specific manner, e.g. as a stand-alone product.

An increasing number of functional food or food supplement products has appeared on the market, claiming to increase disease resistance, yet often based on esoteric arguments rather than scientific evidence. These products are sold without restrictions in supermarkets, and their route of application is usually oral. Their composition can vary from well-defined vitamin or trace element cocktails to complex extracts from organic matter (plants, microorganisms, animals), and includes the so-called probiotics that contain live microorganisms (e.g. Actimel® containing L. casei defensis).

Another category of vaccine-like products such as for example Broncho-Vaxom®, Buccalin® and Uro-Vaxom® are registered medical products. These are either freely available as over the counter ("OTC") medications or as prescribed drugs, and are sold with precise indications, e.g. for the prophylaxis and treatment of infections of the respiratory and lower urinary tract. They are composed of a specific cocktail of inactivated bacteria types which are frequently associated with the respective diseases. In sharp contrast to "real" prophylactic vaccines, the treatment schedule foresees daily oral applications over an extended period of time, both for disease prevention and treatment. The products show protective effects in preclinical models and have documented effects in humans (T cell activation, increased interferon responses and IgA levels), although the mode of action remains unclear.

Baypamun®/Zylexis® (Pfizer) is an example of an injectable immunostimulant for veterinary use. The product contains inactivated ovine parapoxvirus as active ingredient and is recommended for the prevention and treatment of infectious or stress-induced diseases in pets and farm animals. Controlled studies showed efficacy at the level of reduction of clinical symptoms in several species (cattle, horse, cat, dog, pig). The unspecific immunostimulatory effect is ascribed to the induction of cytokines, in particular of interferon. According to the product sheet, the immunostimulatory effect starts a few hours after injection and lasts up to 14 days. These kinetics are ideal for the treatment of infected herds in a situation where a specific immune prophylaxis is too late to be effective. The observed positive effects in animals have led to off-label use of Zylexis^{®} in humans, even against recommendations of health authorities, mostly in cancer patients.

Interferons are widely used as an at least partially effective antiviral treatment (viral hepatitis) that is not pathogen-specific. The recombinant proteins are given i.v. and enhance/mimic one of many effector functions of a natural unspecific response against viral infection. However, the use of interferons is restricted due to severe side effects, high costs and long treatment intervals.

Levamisole (Ergamisol®), a synthetic imidazothiazole derivative, is an antibiotic drug used in combination with fluorouracil to treat colon cancer. It was originally developed and used as an antihelminthic in both humans and animals. Its mechanism of action against worms is well documented. In treating colon cancer the mechanism is documented but completely unclear. Levamisole has been shown to have immunostimulating properties. It is also used infrequently to treat melanoma and head and neck cancer.

WO 2006/085983 discloses the use of viral adjuvants for enhancing the immune response to an immunogen. The viral adjuvants described are replicating, but propagation-defective virus particles containing the viral genome in either modified or unmodified form. A disadvantage of using such viral particles as non-specific immunostimulators is that at least parts of the viral genome must be introduced into the patient with unforeseeable effects. Further, the viral particles introduced into the patient must be replication-competent.

Virosomes are semi-synthetic complexes composed of lipids and at least one viral envelope protein, produced by an in vitro procedure. The lipids are either purified from eggs or plants or produced synthetically, and a fraction of the lipids originates from the virus providing the envelope protein. Essentially, virosomes represent reconstituted, empty virus envelopes, often derived from one or more influenza viruses, devoid of the nucleocapsid including the genetic material of the source virus(es). Virosomes are not able to replicate but are pure fusion-active vesicles. For this reason, virosomes, like liposomes, are typically used to deliver a substance (e.g. an immunogenic molecule, a drug and/or a gene) to a target cell. But unlike liposomes, virosomes offer the advantage of efficient entry into the cells followed by the intracellular release of the virosomal contents triggered by the viral envelope protein. Moreover, due to the incorporation of active viral envelope proteins into their membranes, virosomes release their contents into the cytoplasm immediately after being taken up by the cell, thereby preventing the degradation of the therapeutic substance in the acidic environment of the endosome (US 6040167).

Virosomes have been especially useful in the field of vaccination, where it is desired to stimulate an immune response to an antigen associated with a particular disease or disorder. In such cases, the antigen is typically encapsulated in or bound to the virosome, which then delivers this antigen to the host immune system to be vaccinated. By virtue of the particular antigen delivered, the resulting prophylactic and/or therapeutic is necessarily specific for the disease or disorder with which the antigen is associated.

Virosomes can further be loaded simultaneously with several different B-cell and T-cell epitopes (Pöltl-Frank et al. (1999). Clin. Exp. Immunol. 117, 496; Moreno et al. (1993). J. Immunol. 151, 489) including universal T-helper cell epitopes (Kumar et al. (1992). J. Immunol. 148, 1499-1505) and others known to those of skill in the art. Thus, virosomes are highly effective adjuvants in modern vaccination, possessing superior properties as antigen delivery vehicles and a strong immunogenic potential while concomitantly minimizing the risk of side effects.

Virosomes are functional, in that their membrane fusion activity closely mimics the well-defined low-pH-dependent membrane fusion activity of the intact virus, which is solely mediated by the viral envelope protein. Like viruses, virosomes are rapidly internalized by receptor-mediated endocytosis or opsonization. In contrast to viral systems virosomes are safe, since the infectious nucleocapsid of the virus has been removed. Thus, virosomes represent a promising carrier system for the delivery of a wide variety of different substances, either encapsulated in their aqueous interior or co-reconstituted in their membranes. Co-reconstitution of different receptors within the virosomal membrane, furthermore, allows the targeting of virosomes to different cells or tissues. Virosomes are mainly used as vaccines by adding antigen onto the surface of the virosomes or by encapsulating antigen in the virosomal lumen or into the lumen of liposomes, with which virosomes exert an adjuvant effect.

Virosomes are reconstituted from influenza virus envelopes and use the same cell receptor-mediated endocytosis as their viral counterparts (Hernandez et al. (1996). Annu Rev Cell Dev Biol 12, 627-661). The receptor binding and the membrane fusion activity of influenza virus with endosomes are known to be mediated by the major viral envelope glycoprotein HA (Bungener et al. (2002). J Liposome Res 12, 155-163; Huckriede et al. (2003). Vaccine 21, 925-931). Similar to viral vectors, the mildly acidic pH in the lumen of endosomes triggers the fusion of virosomal with endosomal membranes and thus the release of encapsulated material such as DNA, RNA, or proteins into the cytosol of APCs. Therefore, exogenous antigens encapsulated in virosomes may access the MHC class I pathway without the need of de novo protein synthesis. Not all virosomes are likely to fuse with endosomal membranes, and therefore a fraction is thought to become available for the MHC class II pathway.

Commercially available virosomal vaccines (INFLEXAL^{®} V, EPAXAL^{®}) have been shown to be very efficacious and safe (Glück et al. (1994). Lancet 344, 160-163; Holzer et al. (1996) Vaccine 14, 982-986). The potential of virosomes as a delivery system has been demonstrated for nucleic acids and peptide-based vaccines, e.g., for malaria (Pöltl-Frank et al. (1999). Clin Exp Immunol 117, 496-503). Recent reports also concluded that synthetic peptide vaccines administrated s.c. with virosomes were able to induce a strong CTL immunity (Amacker et al. (2005). Int Immunol 17, 695-704).

WO 2004/045582 describes that, under appropriate in vitro conditions, an empty virosome (i.e. a virosome neither containing nor bearing an antigenic molecule of interest) can be made to fuse with a liposome containing or bearing such a molecule. When the resulting fusogenic particle, containing the antigenic molecule, is administered to a host, the elicited immune response to the antigenic molecule is greater than if this antigenic molecule were to be administered in the liposome alone. Similarly, in EP 05027624.5 it is described that a combination of empty virosomes and liposomes bearing or containing an antigenic molecule, existing as non-fused, separate entities in solution, is capable of potentiating a host immune response to an extent greater than that achieved with the antigen-containing liposome alone.

However, as efficient as virosomes are in their direct delivery of antigenic molecules or adjuvant activity in potentiating the same, virosome activity as described in the prior art is specific in nature, meaning that it manifests itself in the prophylaxis and/or treatment of a specific disease or disorder. The specific disease or disorder prevented and/or treated is dictated by the nature of the antigenic molecule delivered.

In light of the above, there exists a need for further non-specific immunostimulators which do not involve introducing potentially hazardous viral genomes and replicating viral particles into patients, which are safe, which are based on a proven mechanism of action, and which are reliable in their activity.

It is an object of the invention to address this need.

It has now been surprisingly found that a lipid vesicle comprising, in its lipid membrane, at least one viral envelope protein, for example a virosome, may be used to effect a stimulation of the immune system in a manner which is independent of any particular disease. This is surprising, as such lipid vesicles bear no antigenic molecules associated with any particular disease (other than the at least one viral envelope protein) or drugs. Still, the inventors have found that they are effective in eliciting a non-specific protective effect even in the absence of any further agents (e.g. liposomes) associated with such antigenic molecules or drugs.

Accordingly, one aspect of the invention relates to a lipid vesicle comprising, in its lipid membrane, at least one viral envelope protein, for use as a medicament.

Without being bound by theory, the inventors attribute the surprising effect observed to the fact that the lipid vesicles of the invention present the host immune system with pathogen-associated molecular patterns ("PAMPs"). Their presence alerts the immune system via the stimulation of local sensors (toll-like receptors and the like) which then lead to an activation of the innate and/or adaptive immunity via cytokines. As a result, the immune system is brought into a temporary state of alertness, reducing the response time to microbiological threats, increasing the magnitude of the non-specific immediate (innate) response, and enhancing the development of the specific adaptive immunity. Essentially, then, the lipid vesicles of the invention function as an immunological "wake-up call" of sorts to increase the resistance against a broad range of diseases for limited period of time.

As used herein, the term "lipid vesicle" refers to a sphere bounded by a lipid bilayer and defining a lumen with a diameter on the order of 80 - 500 nm. "Lipid vesicle" as used herein refers to a lipid vesicle according to the invention, meaning a lipid vesicle with at least one viral envelope protein in its lipid membrane, and belongs to the class of compounds termed "proteoliposomes".

As used herein, the phrase "in its lipid membrane" refers to a physical attachment of the viral envelope protein to the lipid vesicle via a transmembrane/anchor domain within the protein molecule, or via a covalently linked lipophilic molecule providing the anchor function, or via non-covalent association, either directly with components of the lipid bilayer or with molecules anchored in the lipid bilayer and, as such, is available for binding to a corresponding receptor on a cell with which the lipid vesicle may fuse.

As used herein, the term "viral envelope protein" refers to any protein encoded by an enveloped virus from which the lipid vesicle of the invention is partly or completely derived and that is present in its lipid membrane. In many cases (but not always), viral envelope proteins are part of the outer virion surface and interact with the host organisms, e.g. with receptors on the surface of cells or with soluble molecules. "Viral envelope protein" is not an immunogenic or antigenic molecule associated with any particular disease or disorder. As such, the viral envelope protein of the lipid vesicle of the present invention is not incorporated in order to elicit any kind of specific immune response against this protein, but rather to trigger at least one immediate, non-specific danger signal long before a specific immune response can develop. Pre-existing specific immunity against the viral envelope protein explicitly does not abolish the function of the envelope protein in the sense of the present innovation. Viral envelope proteins sometimes function as "viral fusion proteins", when they play a role in the fusion of viruses or virosomes with target cells.

As used herein, the term "immune response" refers to an increased resistance of an animal to a disease or disorder. An immune response is a physiological response in humans and other higher animals to defend the body against introduction of foreign material and/or its pathological own material (e.g. in the context of cancer, autoimmune disease or disorder).

As used herein, the terms "non-specific", "unspecific" and the like refer to the general immunostimulatory activity of the claimed lipid vesicle, meaning that the immune system is potentiated in its ability to prevent, combat and/or eliminate any one of many diseases or disorders rather than just a single disease or disorder. Conversely, specific immunostimulatory activity refers to the stimulation of the immune system to prevent, combat and/or eliminate a specific disease or disorder. For example, vaccination against a particular disease is an example of eliciting a specific immunostimulatory activity.

As used herein, the terms "therapeutic", "therapy" and the like refer to action taken against a disease or disorder which has already been contracted, or which is suspected of already having been contracted, regardless of whether any corresponding symptoms have already set in. As such, "therapy" and "therapeutic" refer to the elimination or at least amelioration of a disease or disorder in a subject such that, if symptoms are already present, these are mitigated or, if no symptoms are yet present, the onset of such symptoms is lessened in severity or excluded altogether. As used herein, the term "prophylactic", "prophylaxis", "prevent", "prevention" and the like refers to action taken to prevent a subject from contracting a disease, when a subject is not suspected of having contracted the disease in the past, but there exists an expectation that the subject is or will be in danger of contracting a particular disease or disorder in the present or future. Furthermore the terms refer to action taken to prevent a subject from contracting a disease, when a subject has already received a vaccination/immunization, the effect of which, however, is not long-lasting. As such, as used herein, an activity would be properly referred to as "prophylactic" or "preventative" as long as the subject in question does not have any disease symptoms but is expected to possibly contract a particular disease or disorder. An action properly initially referred to as "prophylactic" may become "therapeutic" if it turns out following at least one initial administration that, despite an initial lack of suspicion that a particular disease or disorder exists in a subject, this subject actually has contracted a particular disease or disorder. Conversely, an activity properly initially referred to as "therapeutic" may, if continued beyond curing a particular disease or disorder, become "prophylactic" or "preventative" in nature if there exists no further reason to suspect that the or another disease may be contracted in the future.

As used herein, the term "pharmaceutical" refers to characteristics of compositions and/or medicaments which render them suitable for administration to a living animal, preferably a human.

As used herein, the terms "potentiating", "immunopotentiating", "stimulating", "immunostimulating", "immunostimulatory" and the like are used interchangeably to refer to a compound or enhancing effect on immune functions which may occur through stimulation of immune effector cells and may lead to destruction or clearance of antigen-bearing pathogens or malignancies, and/or to immunity thereto.

As used herein the term "virosome" refers to a reconstituted viral envelope which can be derived from a variety of viruses but which lacks the infectious nucleocapsids and the genetic material of the source virus. A virosome is a special type of lipid vesicle comprising, in its lipid membrane, at least one viral envelope protein. Due to its composition, such structures belong to the class of compounds termed "proteoliposomes". The proteins comprised in the membranes of a proteoliposome are not necessarily derived from enveloped viruses but may originate from any living organism (including microorganisms such as bacteria, fungi, or parasites) or recombinant proteins based on synthetic genes, provided that the biochemical properties of the protein allow its physical attachment to a lipid membrane. Virosomes consist of a mixture of membrane lipids either of viral or non-viral origin and one or more viral envelope proteins. These envelope proteins account for the virosomal functionality, so that their membrane fusion activity closely mimics the well-defined low-pH-dependent membrane fusion activity of the intact virus, which is solely mediated by the viral envelope protein. Like viruses, virosomes are rapidly internalized by receptor-mediated endocytosis. In contrast to viral systems, virosomes are safe, since the infectious nucleocapsid of the virus has been removed. So far, virosomes are mainly used as vaccines by incorporating antigen onto the surface or into the lumen of the virosomes. In contrast to virus-like particles (VLPs), virosomes do not form spontaneously upon recombinant expression of the protein in an appropriate expression system but are the result of a controlled *in vitro* process, which allows large-scale industrial production of virosomes. The resulting virosomes contain a lipid bilayer composed mainly of synthetic lipids, whereas VLPs are made of cellular lipids and, most of the time, form no bilayers. Furthermore, in case of virosomes the content of the single components i.e. lipids, virus proteins can be varied according to the requirements for the final product.

As used herein, the terms "disease" and "disorder" refer to an abnormality of the body or mind that causes discomfort, dysfunction, or distress and is classified into infectious, non-infectious, neoplastic, immune or metabolic disorder or disease.

As used herein, the terms "naked" and "empty" are used interchangeably with reference to lipid vesicles, especially with reference to virosomes, and refer to the fact that the so-characterized vesicles or virosomes contain no disease-specific antigen, nor do they bear any in their lipid bilayer. As such, a "naked" or "empty" lipid vesicle, such as a "naked" or "empty" virosome, contains nothing but the surrounding solution in its lumen, and nothing but the at least one viral envelope protein in its lipid membrane.

A lipid vesicle comprising, in its lipid membrane, at least one viral envelope protein, may advantageously be a virosome.

The preparation of virosomes is well-known by the person skilled in the art. For example, suitable protocols for the preparation of virosomes are described, for example, in EP 538437 or, alternatively, in Mischler and Metcalfe (2002). Vaccine 20, B17-23, incorporated herein by reference.

For example, virosomes may be reconstituted from original viral membrane lipids and spike glycoproteins after solubilization of, for example, intact influenza virus with octaethyleneglycol monododecyl ether, sedimentation of the nucleocapsid (the viral glycoproteins and lipids will remain in the supernatant), and removal of the detergent in the supernatant with a hydrophobic resin (Bio-Beads SM2) (WO 92/19267).

Preparation of virosomes containing HAs from different strains of viruses may be performed with equal amounts of proteins of those viruses solubilized with the non-ionic detergent octaethyleneglycol monododecyl ether. After removal of the detergent with Bio-Beads SM2, virosomes containing different types of envelope proteins may be formed.

An especially preferred form of virosomes is an immunopotentiating reconstituted influenza virosome ("IRIV" or "influenza virosome"). These are spherical, unilamellar vesicles with a mean diameter on the order of 150 nm, prepared from a mixture of phospholipids and influenza virus surface glycoproteins, but they do not contain any viral nucleic acids. The hemagglutinin ("HA") membrane glycoprotein of influenza virus plays a key role in the mode of action of influenza virosomes. This major antigen of influenza virus is a fusion-inducing component, which faciliates antigen delivery to immunocompetent cells.

Influenza virus subtypes from which the at least one viral envelope protein may advantageously be derived are influenza H1N1, influenza H1N2, influenza H2N2, influenza H3N2, influenza H3N8, influenza H5N1, influenza H5N2, influenza H5N3, influenza H5N8, influenza H5N9, influenza H7N1, influenza H7N2, influenza H7N3, influenza H7N4, influenza H7N7, influenza H9N2 and/or influenza H10N7. Further, the at least one viral envelope protein may advantageously be derived from influenza A/Bangkok/1/79, influenza A/Beijing/32/92, influenza A/Brazil/11/78, influenza A/California/7/2004 (H3N2), influenza A/Chile/1/83, influenza A/Christchurch/4/85, influenza A/England/42/72, influenza A/Fujian/411/2002 (H3N2), influenza A/Guizhou/54/89, influenza A/Hong Kong/1/68, influenza A/Johnannesburg/33/94, influenza A/Leningrad/360/86, influenza A/Mississippi/1/85, influenza A/Moscow/10/99 (H3N2), influenza A/New Caledonia/20/99 (H1 N1), influenza A/Panama/2007/99 - RESVIR-17), influenza A/Philippines/2/82, influenza A/Port Chalmers/1/73, influenza A/Scotland/840/74, influenza A/Shangdong/9/93, influenza A/Shanghai/11/87, influenza A/Sichuan/2/87, influenza A/Singapore/6/86, influenza A/Sydney/5/97, influenza A/Texas/1/77, influenza A/USSR/90/77, influenza A/Victoria/3/75, influenza A/Wisconsin/67/2005 (H3N2), influenza A/Wuhan/359/95, influenza A/Wyoming/3/2003 X-147), influenza B/Hong Kong/330/2001, influenza B/Jilin/20/2003, influenza B/Malaysia/2506/2004, influenza B/Shanghai/361/2002, influenza A/Beijing/262/95, influenza B/Victoria/98926/70, influenza B/Singapore/222/79, influenza B/USSR/100/83, influenza B/Yamagata/16/88, influenza B/Panama/45/90, influenza B/Hong Kong/5/72, influenza B/Ann Arbor/1/86, influenza A/Bayern/7/95, influenza B/Shangdong/7/97), and/or B/Jiangsu/10/2003.

Influenza virosomes comprise a double lipid membrane, consisting essentially of phospholipids, preferably phosphatidylcholines (PC) and phosphatidylethanolamines (PE). In contrast to liposomes, influenza virosomes contain the functional viral envelope glycoproteins HA and neuraminidase ("NA") intercalated in the phospholipid bilayer membrane. The biologically active HA not only confers structural stability and homogeneity to virosomal formulations but also significantly contributes to the immunological properties by maintaining the fusion activity of a virus.

Influenza virosomes act as efficient and highly effective means of non-specifically enhancing the immune response. They are also known to have an excellent safety profile (Schaad et al. (2000). Antimicrob Agents Chemother 44, 1163-1167; Glick et al. (2000). J. Infcet. Dis. 181, 1129-1132), meaning that they are well suitable for use in medications intended for unspecific immunostimulation in humans.

Influenza virosomes can be reconstituted from the original viral membrane lipids and spike glycoproteins after solubilization of inactivated influenza virus with octaethyleneglycol monododecyl ether, sedimentation of the nucleocapsid (the viral glycoproteins and lipids will remain in the supernatant), and removal of the detergent in the supernatant with a hydrophobic resin (Bio-Beads SM2). Protocols for the preparation of influenza virosomes are given in WO 92/19267 and for generic virosomes in WO 04/071492.

While it is advantageous that the at least one viral envelope protein be derived from an influenza virus, the lipid vesicle of the present invention may, additionally or alternatively, comprise one or more viral envelope proteins from other types of viruses than influenza. For example, the lipid vesicle may comprise one or more viral envelope protein chosen from, for example, vesicular stomatitis virus (VSV) G protein, Semliki forest virus (SFV) E1 protein, Sendai virus F protein, Respiratory Syncytial Virus (RSV) F- or G-protein or Hepatitis C virus (HCV) E protein.

The lipid vesicle of the present invention may also be a chimeric virosome, meaning that it contains viral envelope proteins, such as hemagglutinin, from at least two different virus strains, for example from influenza strains X-31 and A/Sing or any of the virus strains mentioned above. Additionally, other known viral envelope proteins may be used, such as vesicular stomatitis virus (VSV) G protein, Semliki forest virus (SFV) E1 protein, or Sendai virus F protein, or G protein or F protein from Respiratory syncytial virus (RSV) or Hepatitis C virus (HCV) E protein among many others, to construct chimeric virosomes capable of undergoing sequential and separate fusion events.

The lipid vesicle of the present invention preferably comprises lipids selected from the group consisting of cationic lipids, synthetic lipids, glycolipids, phospholipids cholesterol, or derivatives thereof. Phospholipids comprise preferably phosphatidylcholine, sphingomyelin, phosphatidylethanolamine, phosphatidylserine, phosphatidylglycerol, phosphatidic acid, cardiolipin, and phosphatidylinositol with varying fatty acyl compositions. Cationic lipids are preferably selected from the group consisting of DOTMA (N-[(1-(2,3-dioleyloxy)propyl]-N,N,N-trimethylammonium chloride), DOTAP (N-[1-(2,3-dioleoyloxy)propyl]-N,N,N-trimethylammonium chloride, DODAC (N,N-dioleyl-N,N,-dimethylammonium chloride), DDAB (didodecyldimethylammonium bromide), TC-Chol (cholesteryl N-(trimethylammonioethyl)carbamate chloride), DC-Chol (cholesteryl N-(dimethylammonioethyl)carbamate chloride), or other cationic cholesterol derivatives, and stearylamine or other aliphatic amines and the like. They may be formulated as small unilamellar liposomes in a mixture with PC (phosphatidylcholine). The lipid vesicles of the present invention may preferably comprise egg-derived PC and, more preferably, 1-oleyl-3-palmitoyl-rac-glycero-2-phosphatidylethanolamine.

Preferably, the lipid vesicle of the invention comprises membrane lipids such as phosphatidylcholine, phoshatidylethanolamine, phosphatidylserine, and/or cholesterol derivatives. In the most preferred embodiment, the lipid vesicle further comprises a cationic lipid, for example cationic lipids are preferably selected from the group consisting of DOTMA (N-[(1-(2,3-dioleyloxy)propyl]-N,N,N-trimethylammonium chloride), DOTAP (N-[1-(2,3-dioleoyloxy)propyl]-N,N,N-trimethylammonium chloride, DODAC (N,N-dioleyl-N,N,-dimethylammonium chloride), DDAB (didodecyldimethylammonium bromide), TC-Chol (cholesteryl N-(trimethylammonioethyl)carbamate chloride), DC-Chol (cholesteryl N-(dimethylammonioethyl)carbamate chloride), or other cationic cholesterol derivatives, and stearylamine or other aliphatic amines, DPPE (dipalmitoylphosphatidylethanolamines), DOGS (Dioleoyl-Glycero-Succinate), DOSPA (2,3-dioleoyloxy-N-[2(sperminecarboxamido)ethyl]-N,N-dimethyl-1-propanaminium trifluoroacetate), DOSPER (1,3-dioleoyloxy-2-(6-carboxyspermyl)propylamide), THDOB (N,N,N',N'-tetramethyl-N,N'-bis (2-hydroxyethyl)-2, 3,-dioleoyloxy-1,4- butanediammonium iodide), DOPA (Dioleoyl-sn-Glycero-Phosphate), DOTP (dioctyl tere-phthalate), DOSC (dioleoyl-succinyl-glycerol), DOTB (dioleoyl-e-(4'-trimethylammonio)-butanoyl-sn-glycerol), DOPC (Dioleoyl-sn-Glycero-Phosphocholine) and the like. Especially preferred, the cationic lipid is chosen from cationic cholesterol derivatives such as TC-Chol (cholesteryl N-(trimethylammonioethyl) carbamate) or DC-Chol (cholesteryl N-(dimethylammonioethyl) carbamate).

The membrane of the lipid vesicle of the invention preferably comprises between 1.9 and 37 mol% DC-Chol or TC-Chol, relating to a total lipid content of the membrane. In an especially preferred embodiment, the content of DC-Chol or TC-Chol in the membrane is between 1.9 and 16 mol% of the total lipid content of the membrane. The residual lipid content of the membrane consists preferably of phospholipids, most preferably phosphatidylcholine and phosphytidylethanolamine in a ratio of 4:1. Additionally, the membrane may contain an amount of HA sufficient to guarantee fusion activity of the lipid vesicle.

A co-emulsifying agent may also be used in order to improve the rigidity and/or the sealing of the lipid vesicle. Examples of co-emulsifying agents are cholesterol esters charged or neutral as cholesterol sulphate, derivatives with a sterol backbone, such as derivatives from vegetable origin, for example sitosterol, sigmasterol, and mixtures thereof.

A lipid vesicle according to the invention may for example be obtained by a process analogous to any one of the processes for making DOTAP-containing virosomes disclosed in Examples 1 to 3 and 6 of WO 97/41834, except that DOTAP is replaced by DOSPER and that the DOSPER concentration in the final virosome membrane is properly adjusted as disclosed in WO 97/41834 and, in particular, does not exceed 30% by weight of the total lipid content of the virosome. Basically, a method of preparation of the present lipid vesicles may comprise the following steps:
a) preparing a buffer solution that comprises a non-ionic detergent and that further comprises DOSPER and other lipids and at least one viral envelope protein;
b) adjusting the lipid concentrations to - based on total membrane lipids - 5 to 30% by weight of DOSPER and to a balance of 95 to 70% by weight of said other lipids comprising phosphatidylcholine (PC) or a derivative thereof and optionally phosphatidylethanolamine (PE) and/or cationic lipids other than DOSPER; and
(c) removing the detergent by dialysis or by treating the solution with microcarrier beads, resulting in the formation of said lipid vesicles.

A further aspect of the invention relates to the use of a lipid vesicle comprising, in its lipid membrane, at least one viral envelope protein, for the preparation of a medicament for non-specifically stimulating the immune response of an animal to a disease or disorder. The lipid vesicle according to the inventive use is as already set out hereinabove.

The (at least one) disease or disorder may be an infectious, a non-infectious, a neoplastic, an immune or a metabolic disease or disorder. In one embodiment, the inventive use entails the application of the lipid vesicle of the invention to healthy subjects facing a temporary increased exposure to one or more infectious diseases or disorders, or of (still) healthy subjects immediately following suspected exposure to one or more infectious diseases or disorders but before appearance of symptoms or confirmation of diagnosis. The classification of an action vis a vis a subject as therapeutic or prophylactic is discussed hereinabove.

The inventive use may also be applied to the treatment of one or more already-existing diseases or disorders, optionally as a complementation of specific treatments of such diseases or disorders.

In one embodiment, the at least one infectious disease or disorder may be a viral disease or disorder, a bacterial disease or disorder, a fungal disease or disorder, a parasitic disease, or disorder or a prionic disease or disorder.

The viral infectious disease or disorder may advantageously be chosen from AIDS, AIDS Related Complex, Chickenpox (Varicella), Common cold, Cytomegalovirus Infection, Colorado tick fever, Dengue fever, Ebola haemorrhagic fever, Epidemic parotitis, Genital warts, Hand foot and mouth disease, Hepatitis, Herpes simplex, Herpes zoster, HPV, Influenza (Flu), Lassa fever, Measles, Marburg haemorrhagic fever, Infectious mononucleosis, Mumps, Poliomyelitis, Progressive multifocal leukencephalopathy, Rabies, Rubella, SARS, Smallpox (Variola), Viral encephalitis, Viral gastroenteritis, Viral meningitis, Viral pneumonia, West Nile disease, Yellow fever. These and other viral infectious diseases or disorders are caused by RSV, Polioviruses, Rubella virus, Dengue virus, Flaviviridae, Coronaviridae, Reoviridae, Rabies virus, Paramyxoviridae (e.g., mumps virus, measles virus, respiratory syncytial virus, etc.), orthomyxoviridae (e.g., influenza virus types A, B and C, etc.), simian immunodeficiency virus (SIV), HAV, HBV, HCV, HDV, HEV, HPV, HSV, HIV, CMV, EBV, Polio virus, varicella, Bunyavirus (e.g. La Crosse virus).

The bacterial infectious disease or disorder may advantageously be chosen from Anthrax, Bacterial Meningitis, Brucellosis, Campylobacteriosis, Cat Scratch Disease, Cholera, Diphtheria, Epidemic Typhus, Gonorrhea, Impetigo- Legionellosis, Leprosy (Hansen's Disease), Leptospirosis, Listeriosis, Lyme Disease, Melioidosis, MRSA infection, Nocardiosis, Pertussis (Whooping Cough), Plague, Pneumococcal pneumonia, Psittacosis, Q fever, Rocky Mountain Spotted Fever (RMSF), Salmonellosis, Scarlet Fever, Shigellosis, Syphilis, Tetanus, Trachoma, Tuberculosis, Tularemia, Typhoid Fever, Typhus; Urinary Tract Infections. These and other bacterial infectious diseases or disorders are caused by Corynebacterium diphtheriae, Clostridium tetani, Bordetella pertussis, Neisseria meningitidis, including serotypes Meningococcus A, B, C, Y and W135, Haemophilus influenza type B (Hib), and Helicobacter pylori.

The fungal infectious disease or disorder may advantageously be chosen from Aspergillosis, Blastomycosis, Candidiasis, Coccidioidomycosis, Cryptococcosis, Histoplasmosis, Tinea pedis.

The parasitic infectious disease or disorder may advantageously be chosen from African trypanosomiasis, Amebiasis, Ascariasis, Babesiosis, Chagas Disease, Clonorchiasis, Cryptosporidiosis, Cysticercosis, Diphyllobothriasis, Dracunculiasis, Echinococcosis, Enterobiasis, Fascioliasis, Fasciolopsiasis, Filariasis, Free-living amebic infection, Giardiasis, Gnathostomiasis, Hymenolepiasis, Isosporiasis, Kala-azar, Leishmaniasis, Malaria, Metagonimiasis, Myiasis, Onchocerciasis, Pediculosis, Pinworm Infection, Scabies, Schistosomiasis, Taeniasis, Toxocariasis, Toxoplasmosis, Trichinellosis, Trichinosis, Trichuriasis, Trypanosomiasis.

The prionic infectious disease or disorder may advantageously be chosen from transmissible spongiform encephalopathy, Bovine spongiform encephalopathy, Creutzfeldt-Jakob disease, Kuru.

The neoplastic disease or disorder may be a cancer. The cancer may for example be a sarcoma, a leukemia, a lymphoma, a myeloma, a melanoma, an adenoma, a carcinoma, a choriocarcinoma, a gastrinoma, a pheochromocytoma, a prolactinoma, or a neuroma.

Specifically, the cancer may be advantageously selected from Adult Acute Lymphoblastic Leukemia; Childhood Acute Lymphoblastic Leukemia; Acute Myeloid Leukemia; Adrenocortical Carcinoma; Childhood Adrenocortical Carcinoma; AIDS-Related Cancers; AIDS-Related Lymphoma; Anal Cancer; Basal Cell Carcinoma; Extrahepatic Bile Duct Cancer; Bladder Cancer; Bone Cancer, Osteosarcoma/Malignant Fibrous Histiocytoma; Brain Stem Glioma; Adult Brain Tumor; Brain Cancer (e.g. Brain Stem Glioma; Cerebellar Astrocytoma; Cerebral Astrocytoma/Malignant Glioma; Ependymoma; Medulloblastoma; Supratentorial Primitive Neuroectodermal Tumors; Visual Pathway and Hypothalamic Glioma); Breast Cancer (female and male); Bronchial Adenomas/Carcinoids; Burkitt's Lymphoma; Carcinoid Tumor; Gastrointestinal Carcinoid Tumor; Carcinoma of Unknown Primary Origin; Primary Central Nervous System Lymphoma; Cervical Cancer; Childhood Cancers; Chronic Lymphocytic Leukemia; Chronic Myelogenous Leukemia; Chronic Myeloproliferative Disorders; Colon Cancer; Colorectal Cancer; Cutaneous T-Cell Lymphoma, Mycosis Fungoides and Sézary Syndrome; Endometrial Cancer; Ependymoma; Esophageal Cancer; Ewing's Family of Tumors; Extracranial Germ Cell Tumor; Extragonadal Germ Cell Tumor; Extrahepatic Bile Duct Cancer; Eye Cancers (e.g. Intraocular Melanoma Eye Cancer and Retinoblastoma); Gallbladder Cancer; Gastric (Stomach) Cancer;; Gastrointestinal Carcinoid Tumor; Gastrointestinal Stromal Tumor (GIST); Extracranial Germ Cell Tumor; Extragonadal Germ Cell Tumor; Ovarian Germ Cell Tumor; Gestational Trophoblastic Tumor; Gliomas; (e.g. Brain Stem Glioma, Cerebral Astrocytoma, Visual Pathway and Hypothalamic Glioma); Hairy Cell Leukemia; Head and Neck Cancer; Primary Hepatocellular (Liver) Cancer; Hodgkin's Lymphoma; Hypopharyngeal Cancer; Intraocular Melanoma; Pancreatic cancer (Islet Cell Carcinoma); Kaposi's Sarcoma; Kidney (Renal Cell) Cancer; Laryngeal Cancer; Acute Leukemias (e.g. Lymphoblastic Leukemia, Acute Myeloid Leukemia, Chronic Lymphocytic Leukemia, Chronic Myelogenous Leukemia, Hairy Cell Leukemia); Lip and Oral Cavity Cancer; Liver Cancer (primary and metastatic); Non-Small Cell Lung Cancer; Small Cell Lung Cancer; AIDS-Related Lymphoma; Burkitt's Lymphoma; Cutaneous T-Cell Lymphoma; Hodgkin's Lymphoma; Non-Hodgkin's Lymphoma; Primary Central Nervous System Lymphoma; Macroglobulinemia, Waldenström's; Malignant Fibrous Histiocytoma of Bone/Osteosarcoma; Medulloblastoma; Melanoma;, Intraocular (Eye) Melanoma; Merkel Cell Carcinoma; Mesothelioma; Primary Metastatic Squamous Neck Cancer with Occult; Multiple Endocrine Neoplasia Syndrome; Multiple Myeloma/Plasma Cell Neoplasm; Mycosis Fungoides; Myelodysplastic Syndromes; Myelodysplastic/Myeloproliferative Diseases; Chronic Myelogenous Leukemia; Myeloid Leukemia; Multiple Myeloma; Chronic Myeloproliferative Disorders; Nasal Cavity and Paranasal Sinus Cancer; Nasopharyngeal Cancer; Nasopharyngeal Cancer; Neuroblastoma; Oropharyngeal Cancer; Osteosarcoma/Malignant Fibrous Histiocytoma of Bone; Ovarian Cancer; Ovarian Epithelial Cancer; Ovarian Germ Cell Tumor; Ovarian Low Malignant Potential Tumor; Pancreatic Cancer; Islet Cell Pancreatic Cancer; Paranasal Sinus and Nasal Cavity Cancer; Parathyroid Cancer; Penile Cancer; Pheochromocytoma; Pineoblastoma and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Primary Central Nervous System Lymphoma; Prostate Cancer; Rectal Cancer; Transitional Cell Cancer of Renal Pelvis and Ureter; Retinoblastoma; Rhabdomyosarcoma; Salivary Gland Cancer; Ewing's Family of Tumors; Soft Tissue Sarcoma; Uterine Sarcoma; Sézary Syndrome; Skin Cancer (non-Melanoma); Skin Cancer (Melanoma); Skin Carcinoma, Merkel Cell; Small Cell Lung Cancer; Small Intestine Cancer; Soft Tissue Sarcoma; Metastatic Squamous Cell Carcinoma; Squamous Neck Cancer with Primary Occult; Stomach (Gastric) Cancer; Supratentorial Primitive Neuroectodermal Tumors; Cutaneous T-Cell Lymphoma; Testicular Cancer; Thymoma; Thymoma and Thymic Carcinoma; Thyroid Cancer; Gestational Trophoblastic Tumor,; Carcinoma of Unknown Primary Site; Cancer of Unknown Primary Site; Unusual Childhood Cancers; Urethral Cancer; Endometrial Uterine Cancer; Uterine Sarcoma; Vaginal Cancer; Vulvar Cancer; Waldenström's Macroglobulinemia; Wilms' Tumor; and Women's Cancers.

According to a further embodiment the animal is a mammal. The mammal is preferably a human, a chimpanzee, a cynomologous monkey, a gibbon, a simian monkey, a macaque monkey, a mouse, a rat, a cat, a dog, a horse, a rabbit, a camel, a llama, a ruminant, a horse or a pig. A preferred ruminant may be a cow, a bull, a goat, a sheep, a bison, a buffalo, a deer or a stag.

In a further embodiment, the medicament is suitable for administration intramuscularly, intradermally, intraveneously (e.g. by injection), subcutaneously, intraperitoneally, parenterally, topically, endotracheally, intraauracularly, intraarticularly, intraocularly, locally, by gargling, by a patch (for example a skin patch), by spray (for example a nasopharyngeal spray) by sublingually, orally (e.g. tablets, capsules, caplets, dragees), by suppository (e.g. rectal suppository or vaginal suppository), or by drops (e.g. eye drops). Administration may be in a single dose or, as need dictates, in multiple doses with intervening time intervals as deemed appropriate by the supervising clinician.

Repeated applications of the lipid vesicles of the invention are conceivable. The combination of the lipid vesicles of the invention with other compounds e.g. adjuvants or immunostimulants may synergistically enhance the overall effect. The amount and type of lipid vesicle, the site of stimulation, and co-stimulating signals (infections, exposure to allergens, etc.) define the overall effect. The effect is transient, on the order of hours to weeks. The duration of the effect achieved depends on dose magnitude, dose timing, the route of administration chosen as well as the composition of the medicament administered.

The medicament prepared according to the inventive use is administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines and optionally other therapeutic agents. The preferred amount of lipid vesicle to be administered depends on the disease or disorder to be treated or prevented. Generally, doses ranging from about 1 ng/kg to about 100 mg/kg are believed to be effective, said kilograms referring to body weight of the animal treated. The preferred range is believed to be from about 10 ng/kg to about 10 µg/kg. The absolute amount will depend upon a variety of factors, including the composition selected for administration, whether the administration is in single or multiple doses, and individual patient parameters including age, physical condition, size, weight, and the stage of the disease.

The route and regimen of administration will vary depending upon the stage or severity of the disease or disorder to be treated, and is to be determined by the skilled practitioner. The medicament prepared by the inventive use is suitable for parenteral administration. Here, the medicament comprises lipid vesicles dissolved or suspended in an acceptable carrier, preferably an aqueous carrier. A variety of aqueous carriers may be used, e.g. water, buffered water, 0.4% saline, 0.3% glycine, hyaluronic acid and the like. These compositions may be sterilized by conventional, well known sterilization techniques, or may be sterile-filtered. The resulting aqueous solutions may be packaged for use as they are, or lyophilized, the lyophilized preparation being combined with a sterile solution prior to administration.

The medicament prepared by the inventive use may additionally contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, among many others. Actual methods for preparing parenterally administrable compounds will be known or apparent to those skilled in the art and are described in more detail in for example, Remington: The Science and Practice of Pharmacy ("Remington's Pharmaceutical Sciences") Gennaro AR ed. 20th edition, 2000: Williams & Wilkins PA, USA, which is incorporated herein by reference.

The medicament prepared according to the inventive use can also be administered in such oral dosage forms for example as tablets, capsules (each including timed release and sustained release formulations), pills, powders, granules, elixirs, tinctures, solutions, suspensions, syrups and emulsions, or by injection. For instance, for oral administration in the form of a tablet or capsule, the active drug component can be combined with an oral, non-toxic pharmaceutically acceptable inert carrier such as ethanol, glycerol, water and the like.

Similarly, the medicament prepared according to the inventive use may also be administered intravenously (either by bolus or infusion methods), intraperitoneally, subcutaneously, topically with or without occlusion, or intramuscularly. In preferred embodiments, the medicament prepared according to the inventive use is administered intramuscularly, subcutaneously, intradermally, mucosal or transdermally. All of these forms are well known to those of ordinary skill in the pharmaceutical arts.

The dosage regimen according to which the medicament prepared according to the inventive use is to be administered is selected in accordance with a variety of factors, including for example species, age, weight, sex and medical condition of the patient, the stage and severity of the disease or disorder to be treated and the particular type of lipid vesicle employed. A physician of ordinary skill in the art can readily determine and prescribe the effective amount of the medicament required to prevent, counter, or arrest the progress of a malignancy or infectious disease or disorder. Optimal precision in achieving concentration of drug with the range that yields efficacy either without toxicity or with acceptable toxicity requires a regimen based on the kinetics of the lipid vesicle's availability to target sites. This process involves a consideration of the distribution, equilibrium, and elimination of the lipid vesicle, and is within the ability of the skilled practitioner and can be addressed with no more than routine experimentation.

In one embodiment, the medicament prepared according to the inventive use may be administered in a single daily dose, or the total daily dosage may be administered in divided doses for example of two, three, or four times daily. In another embodiment, weekly or monthly administrations are foreseen.

The daily dose the medicament prepared according to the inventive use may be varied over a range from 10 ng/kg to about 10 µg/kg of lipid vesicles per adult per day. For oral administration, the medicament prepared according to the inventive use is preferably provided in the form of tablets containing from 0.001 to 1,000 mg, preferably 0.001, 0.01, 0.05, 0.1, 0.5, 1, 2.5, 10, 20, 50, 100 milligrams of lipid vesicle for the symptomatic adjustment of dosage according to signs and symptoms of the patient in the course of treatment. An effective amount of lipid vesicle in the medicament prepared according to an embodiment the inventive use is ordinarily supplied at a dosage level of from about 0.0001 mg/kg to about 50 mg/kg of body weight per day. More particularly, the range is from about 0.0001 mg/kg to 7 mg/kg of body weight per day.

Furthermore, the medicament prepared according to the inventive use can be administered in intranasal form, or via transdermal routes known to those of ordinary skill in the art. To be administered in the form of a transdermal delivery system, the administration dosage will be continuous rather than intermittent throughout the dosage regimen.

The medicament prepared according to the inventive use may be coupled to a class of biodegradable polymers useful in achieving controlled release of a drug, for example, polylactic acid, polyepsilon caprolactone, polyhydroxy butyric acid, polyorthoesters, polyacetals, polydihydro-pyrans, polycyanoacrylates, and cross-linked or amphipathic block copolymers of hydrogels.

A suitable formulation of the medicament prepared according to the inventive use for topical administration may be, for example, in the form of a solution, cream, ointment, gel, lotion, shampoo, or aerosol formulation adapted for application to the skin. These topical pharmaceutical composition containing the medicament prepared according to the inventive use ordinarily include about 0.005% to 5% by weight of the active compound, i.e. the lipid vesicle, in admixture with a pharmaceutically acceptable vehicle.

Regardless of the route by which the medicament prepared according to the inventive use is administered, it is to be administered in an effective amount. An effective amount is that amount of a pharmaceutical preparation that, alone or together with further doses, stimulates the desired non-specific immunostimulatory response.

Moreover, when desired or necessary, suitable binders, lubricants, disintegrating agents and coloring agents can also be incorporated into the medicament as prepared by the inventive use. Suitable binders include, without limitation, starch, gelatin, natural sugars such as glucose or beta-lactose, corn sweeteners, natural and synthetic gums such as acacia, tragacanth or sodium alginate, carboxymethyl cellulose, polyethylene glycol, waxes and the like. Lubricants used in these dosage forms include, without limitation, sodium oleate, sodium stearate, magnesium stearate, sodium benzoate, sodium acetate, sodium chloride and the like. Disintegrators include, without limitation, starch, methylcellulose, aga, bentonite, xanthan gum and the like.

The liquid forms of the medicament as prepared by the inventive use may be suitably flavored by suspending or dispersing agents such as the synthetic and natural gums, for example, tragacanth, acacia, methyl cellulose and the like. Other dispersing agents, which may be employed, are glycerin and the like. For parenteral administration, sterile suspensions and solutions are desired. Isotonic preparations, which generally contain suitable preservatives, are employed when intravenous administration is desired. Topical preparations containing the active drug component can be admixed with a variety of carrier materials well known in the art, such as, for example, alcohols, aloe vera gel, allatoin, glycerin, vitamins A or E oils, mineral oil, PPG2 myristoyl propionate, and the like, to form, for example, alcoholic solutions, topical cleansers, cleansing creams, skin gels, skin lotions, and shampoos in cream or gel formulations.

In one embodiment, the medicament as prepared by the inventive use may further comprise at least one adjuvant enhancing and/or mediating an immune response, for example an innate immune response, a Th₁ or Th₂ response. Suitable adjuvants may enhance the immunological response by activating macrophages and/or stimulating specific sets of lymphocytes. A suitable adjuvant may be any ligand suitable for the activation of a pathogen recognition receptor (PRR). Immune response-potentiating compounds are classified as either adjuvants or cytokines. Adjuvants may enhance the immunological response by providing a reservoir of antigen (extracellularly or within macrophages), activating macrophages and stimulating specific sets of lymphocytes.

Adjuvants of many kinds are well known in the art; specific examples include Freund's (complete and incomplete), mycobacteria such as BCG, M. vaccae, or Corynebacterium parvum, Cholera toxin or tetanus toxin, E.coli heat-labile toxin, quil-saponin mixtures such as QS-21 (SmithKline Beecham), MF59 (Chiron) and various oil/water emulsions (e.g. IDEC-AF). Other adjuvants which may be used include, but are not limited to: mineral salts or mineral gels such as aluminum hydroxide, aluminum phosphate, and calcium phosphate; surface active substances such as lysolecithin, pluronic polyols, polyanions, peptides, keyhole limpet hemocyanins, and dinitrophenol, immunostimulatory molecules, such as saponins, muramyl dipeptides and tripeptide derivatives, short nucleic acid stretches such as CpG dinucleotides, CpG oligonucleotides, monophosphoryl Lipid A, and polyphosphazenes, particulate and microparticulate adjuvants, such as emulsions, liposomes, virosomes, cochleates, or immunostimulating complex adjuvants.

Cytokines are also useful due to their lymphocyte stimulatory properties. Many cytokines useful for such purposes will be known to one of ordinary skill in the art, including interleukin-2 (IL-2), IL-12, GM-CSF and many others. Furthermore ligands from the chemokine family, such as RANTES (Regulated upon Activation Normal T cell Expressed and Secreted), a lipoprotein of Gram-positive bacteria, a yeast cell wall component, a double-stranded RNA, a lipopolysaccharide of Gram-negative bacteria, flagellin, a U-rich single-stranded viral RNA, , a Suppressor 6f Cytokine Signalling small interfering RNA (SOCS siRNA), a Pan DR epitope (PADRE) and mixtures thereof are suitable.

For treatment and prevention of cancers and/or metastases, the medicament as prepared by the inventive use may be administered in combination with a pharmaceutically acceptable carried adopted for topical administration. Additionally, for the treatment and prevention of cancer, tumors and/or metastases, or viral infections, the medicament as prepared by the inventive use may be used together with other agents known to be useful in treating such malignancies. For combination treatment with more than one active agent, where the active agents can be administered concurrently, the active agents can be administered concurrently, or they can be administered separately at staggered times.

A further aspect of the invention relates to a method of non-specifically stimulating the immune response of an animal to a disease, comprising administering a lipid vesicle comprising, in its lipid membrane, at least one viral envelope protein to an animal.

### Brief description of the figures

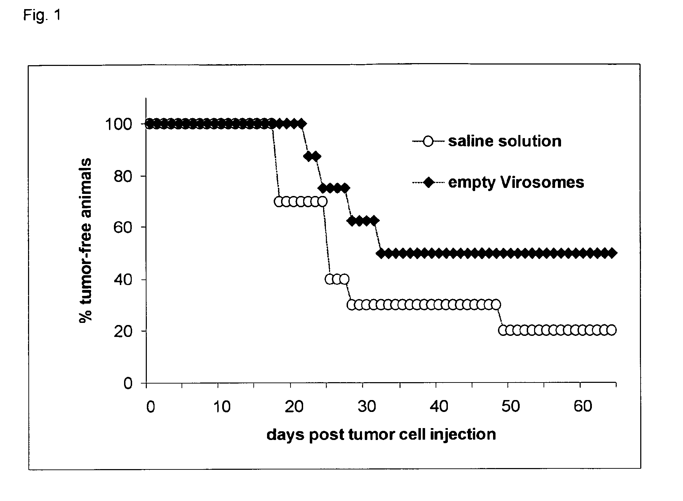

- Fig. 1: Effect of naked virosomes on tumor growth in mice. The figure shows the results of monitoring tumor growth in mice which had been injected with tumor cells and, subsequently, weekly with one of two samples: saline solution as a control (open circles); and empty virosomes (solid diamonds).
The results show that injection with naked, i.e. empty virosomes resulted in significantly more tumor-free mice at the end of the experimental period than injection with the control.

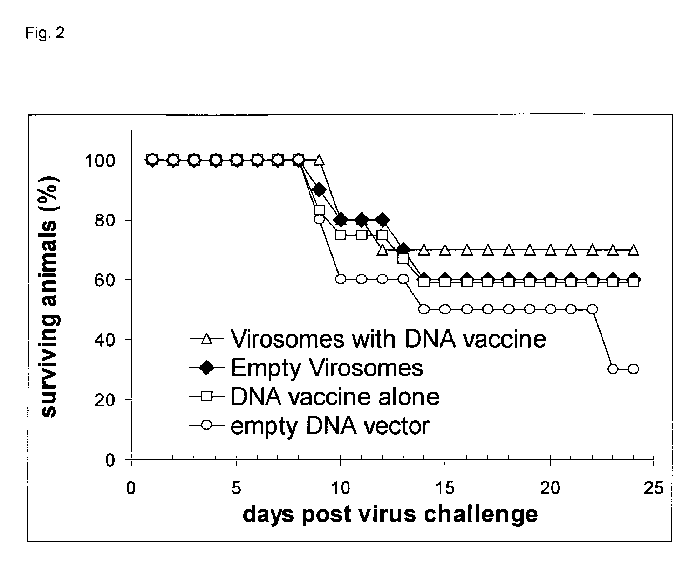

- Fig. 2: Protective effect of naked virosomes on progression of La Crosse virus. The figure shows the results of monitoring the survival of mice challenged with La Crosse virus. The mice had been injected with one of four samples prior to viral challenge: virosomes encapsulating DNA encoding La Crosse virus glycoproteins (open triangles); empty virosomes (solid diamonds); DNA vector alone, said vector comprising DNA expressing La Crosse virus glycoproteins (open squares); DNA vector alone, said vector lacking DNA expressing La Crosse virus glycoproteins (open circles). The results indicate that treatment with naked virosomes resulted in an improved survival rate as compared to the DNA vector control lacking DNA expressing La Crosse virus glycoproteins.

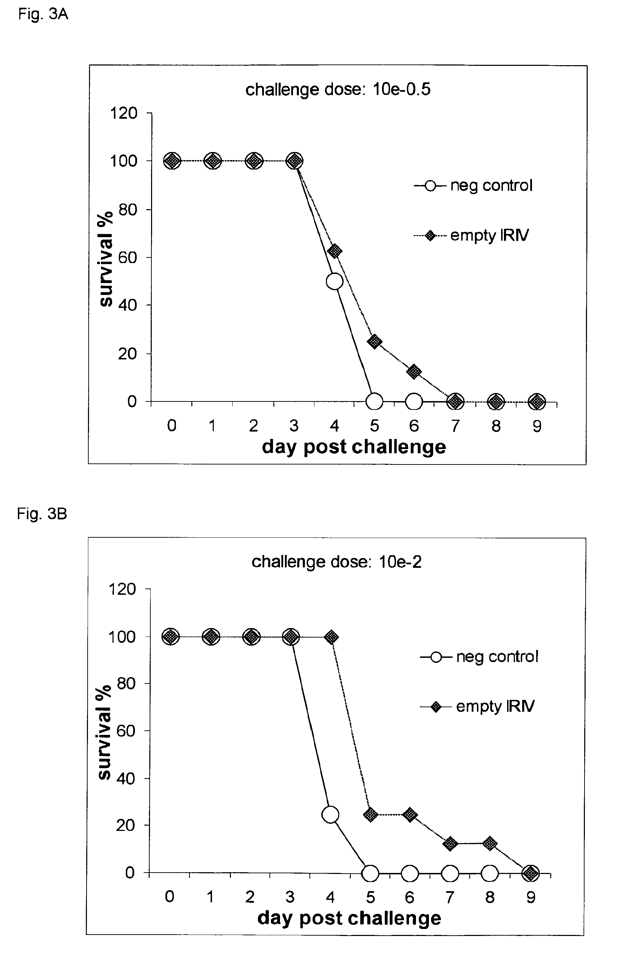

- Figs. 3A, 3B: Protective effect of empty virosomes on progression of Leptospira interrogans infection. The figure shows the result of monitoring the survival rate of gerbils challenged with the bacterium Leptospira interrogans. The gerbils had been injected with empty influenza virosomes (shaded diamonds) prior to bacterial challenge. As a negative control "untreated" gerbils were challenged (open circles).

The specific embodiments of the invention and the following examples are provided to demonstrate the efficiency of the claimed invention but are not to be interpreted as limiting the scope of the invention. To the extent that specific materials are mentioned, it is merely for purposes of illustration and is not intended to limit the invention. Unless otherwise specified, biochemical and molecular biology procedures, such as those set forth in Voet, Biochemistry, Wiley, 1990; Stryer, Biochemistry, W.H. Freeman, 1995; Bodanszky, Peptide Chemistry. A Practical Textbook, 2nd ed., Springer-Verlag, Berlin, 1993; Sambrook et al., Molecular Cloning, Cold Spring Harbor Laboratory, 2001; Ausubel et al. (Eds.), Current Protocols in Molecular Biology, John Wiley & Sons, 2000 are used. One skilled in the art may develop equivalent means or reactants without the exercise of inventive capacity and without departing from the scope of the invention.

It will be understood that many variations can be made in the compositions and procedures herein described while still remaining within the bounds of the present invention. It is the intention of the inventors that such variations are included within the scope of the invention.

### Example 1 (preparation example): Reagents used in preparation and working examples

Reagents: Octaethyleneglycol-mono-(n-dodecyl)ether (OEG, C₁₂E₈), is purchased from Fluka Chemie GmbH-(Buchs, Switzerland). Sucrose (Eur. Phar.) is purchased from Merck (Dietikon, Switzerland). Egg phosphatidyl choline (PC) is obtained from Lipoid (Cham, Switzerland). 1-Oleoyl-3-palmitoyl-rac-glycero-2-phosphoethanolamine is obtained from Bachem (Bubendorf, Switzerland). Bio-Beads SM2 are purchased from Bio-Rad Laboratories (Glattbrugg, Switzerland). Cholesteryl N-(trimethylammonioethyl)carbamate chloride (TC-chol) was purchased from Merck Eprova (Schaffhausen, Switzerland).

Influenza viruses of the A/Singapore/6/86 (A/Sing) strain and other influenza A strains, propagated in the allantoic cavity of embryonated eggs (Gerhard, W. (1976), J. Exp. Med. 144:985-995), were obtained from Berna Biotech AG (Bern, Switzerland) and purified as described (Skehel, J. et al.,(1971). Virology 44:396). The hemagglutinin/phospholipid ratio was determined according to Böttcher (Böttcheret al. (1961). Anal. Chim. Acta 24, 203) and HA-quantification after SDS-PAGE with the Coomassie-extraction method as described by Ball (Ball (1986). Anal. Biochem. 155, 23).

### Example 2 (preparation example): Preparation of the standard virosomes as influenza virosomes

For a final volume of 4 ml, 32 mg egg PC and 8 mg OPPE are dissolved in 3 ml of PBS, 100 mM OEG (PBS/OEG). 2 mg HA of inactivated influenza A/Singapore/6/86 virus or another influenza A strain is centrifuged at 100,000 x g for 1 h at 4°C and the pellet is dissolved in 1 ml of PBS/OEG. The detergent solubilized phospholipids and viruses are mixed and sonicated for 1 min. This mixture is centrifuged at 100,000 x g for 1 h at 18°C. Virosomes are formed by detergent removal using two times 1.5 g of wet SM2 Bio-Beads for 1 h each at room temperature with shaking. The freshly formed virosomes are then sterile filtered (0.22 µm).

### Example 3 (preparation example): Preparation of virosomes containing TC-Chol

Virosomes containing TC-Chol are prepared by the detergent removal method. For a final volume of 4 ml, 32 mg egg PC, 8 mg OPPE and 5 mg cholesteryl N-(trimethylammonioethyl)carbamate chloride (TC-chol) are dissolved in 2.6 ml of PBS, 100 mM OEG (PBS/OEG). 2 mg HA of inactivated A/Singapore/6/86 influenza virus or another influenza A strain is centrifuged at 100,000 x g for 1 h at 4°C and the pellet is dissolved in 1 ml of PBS/OEG. The detergent solubilised phospholipids and viruses are mixed with 0.4 mL of 50% (w/v) sucrose and sonicated for 1 min. This mixture is centrifuged at 100,000 x g for 1 h at 18°C. Virosomes are formed by detergent removal using two times 1.5 g of wet SM2 Bio-Beads for 1 h each at room temperature with shaking. The freshly formed virosomes are then sterile filtered (0.22 µm) and aliquoted in sterile glas vials. The closed vials are frozen at -70°C and then lyophilized at -40°C for 20 h and 10°C for 2 h. The closed vials are stored at frozen until use.

### Example 4 (working example): Non-specific immunostimulation with emptyvirosomes decreases tumor burden in mice

The following experiment was performed in the context of the development of a cancer vaccine for therapeutic use. The vaccine is intended to be applied while the tumor is already developing in the body. It is intended that immunization with virosomes enhances the specific immune response against the tumor to a level sufficient for complete elimination of the growing tumor cells.

Eight to ten week-old female C57BI/6 mice were pre-immunized with inactivated influenza virus containing 1 µg hemagglutinin to simulate the situation in pre-immunized humans who have become naturally infected with influenza at some previous time in life. Three weeks later, 10,000 B16 tumor cells were injected subcutaneously. Two days later, immunization with approx. 1-25 mg empty virosomes per kg body weight were started and continued at weekly intervals for 9 weeks.

The experiment included two treatment groups. Mice in each respective group were treated as follows:
Group 1: Phosphate-buffered saline ("saline solution" in Fig.1; open circles)
Group 2: Standard (empty) virosomes composed of egg-derived phospholipids and solubilized influenza envelopes ("empty virosomes" in Fig.1; solid diamonds)

During the treatment period, the animals were closely monitored for the development of tumors. Mice that had developed palpable tumors were euthanized. Accordingly, the readout of the study was the number of tumor-free mice per group throughout the observation period. The results are shown in Fig. 1. As can be taken from Fig.1, 50% of the mice treated with empty virosomes were significantly protected (solid diamonds in Fig.1; "empty virosomes"), while only 20% of the mice treated with saline control remained tumor-free (open circles in Fig. 1; "saline solution").

In conclusion, administration of naked virosomes led to the eradication of tumors in a significant number of tumor-burdened mice.

### Example 5 (working example): Non-specific immunostimulation with naked virosomes increases survival rate in mice infected with La Crosse virus

The experiment was originally performed to assess virosomes as a delivery vehicle for a DNA-based vaccine against La Crosse virus. Plasmid DNA encoding viral glycoproteins of LaCrosse virus (VR1012-G1 G2) is packaged into virosomes in order to increase plasmid uptake and expression of viral antigens. One of the administered controls was naked virosomes.

Genetically modified mice lacking the receptor for type I interferons (IFNAR-I -/- mice) are more susceptible to viral infections and are thus a suitable challenge model for La Crosse virus. The mice were pre-immunized against influenza to simulate the situation in pre-immunized humans, who have become naturally infected with influenza at some previous time in life, and a single dose of the prototype La Crosse vaccine (or control formulations as indicated below) was administered ten days later. The challenge with live virus at a dosage of 200,000 infectious units was performed 4 weeks later. The survival of the animals was monitored for 24 days after infection. The following groups were included in the experiment:
1. DNA vector expressing La Crosse glycoproteins combined with Virosomes ("Virosomes with DNA vaccine" in Fig. 2; open triangles)
2. Virosomes without DNA ("Empty Virosomes" in Fig. 2; solid diamonds)
3. DNA vector alone, the vector expressing La Crosse virus glycoproteins ("DNA vaccine alone" in Fig. 2; open squares)
4. DNA vector alone, the vector not expressing La Crosse virus glycoproteins ("empty DNA vector" in Fig. 2; open circles)

The results of the study are shown in Fig. 2. As can be taken from Fig. 2, empty virosomes provided equally good protection as the DNA vector expressing La Crosse virus glycoproteins when administered alone. In each case, 60% of the animals were protected. In comparison, the group receiving DNA vector plus virosomes showed a protection rate of 70%. As a negative control, the DNA vector NOT expressing La Crosse virus glycoproteins was administered. This still resulted in a protection rate of 30%.

The three week interval between immunization and challenge may be necessary for the development of adaptive immunity, but it is likely suboptimal to assess a non-specific protective effect manifested by naked virosomes. Despite this suboptimal administration, however, while not as effective as virosomes encapsulating La Crosse-derived DNA, initial immunization with naked (i.e. empty) virosomes still elicited a non-specific immunostumulatory effect sufficient to improve survival rate by about 30% as compared with the control.

### Example 6 (working example): Non-specific immunostimulation with naked virosomes increases survival rate in gerbils infected with the bacterium Leptospira interrogans

The experiments were performed in the context of the development of a prophylactic vaccine against Leptospira interrogans. Two different challenge doses of Leptospira interrogans were used. Virosomes were prepared as outlined in example 2 ("empty influenza virosomes").

Gerbils were immunized two weeks prior to challenge with live bacteria. The challenge was performed with different dosages of the pathogen, as indicated in the separate Figs. 3A and 3B.
1. negative control: untreated animals ("neg control" in Figs. 3A and 3B; open circles)
2. empty virosomes (empty influenza virosomes" in Figs. 3A and 3B; shaded diamonds)

The results are shown in Figs. 3A and 3B. As can be taken from Figs. 3A and 3B, while none of the animals survived the bacertial infection, animals which had been pre-treated with empty virosomes survived longer than the untreated control animals.

## Claims

1. A lipid vesicle comprising, in its lipid membrane, at least one viral envelope protein, for use as a medicament.

2. The lipid vesicle of claim 1, wherein the at least one viral envelope protein is an influenza virus envelope protein.

3. The lipid vesicle of claim 2, wherein the at least one viral envelope protein is hemagglutinin (HA) and/or neuraminidase (NA).

4. The lipid vesicle of claim 2, wherein the viral envelope proteins are hemagglutinin (HA) and neuraminidase (NA).

5. The lipid vesicle of claim 1, wherein the at least one viral envelope protein is a viral envelope protein selected from
• G protein of vesicular stomatitis virus (VSV);
• E1 protein of Semliki forest virus (SFV); and/or
• F protein of Sendai virus
• E-protein of Hepatitis C virus (HCV)
• F-protein of Respiratory syncytial virus (RSV)
• G-protein of Respiratory syncytial virus (RSV)

6. The lipid vesicle of any of the above claims, wherein the lipid vesicle is a virosome.

7. The lipid vesicle of claim 6, wherein the virosome is an immunopotentiating reconstituted influenza virosome ("influenza virosome"), or a chimeric virosome.

8. The lipid vesicle of claim 7, wherein the influenza virosome or the chimeric virosome is a chimeric influenza virosome.

9. Use of a lipid vesicle comprising, in its lipid membrane, at least one viral envelope protein, for the preparation of a medicament for non-specifically stimulating the immune response of an animal to a disease or disorder.

10. The use of claim 9, wherein the at least one viral envelope protein is an influenza virus envelope protein.

11. The use of claim 10, wherein the at least one viral envelope protein is hemagglutinin (HA) or neuraminidase (NA).

12. The use of claim 10, wherein the viral envelope proteins are hemagglutinin (HA) and neuraminidase (NA).

13. The use of claim 9, wherein the at least one viral envelope protein is a viral envelope protein selected from
• G protein of vesicular stomatitis virus (VSV);
• E1 protein of Semliki forest virus (SFV); and/or
• F protein of Sendai virus
• E-protein of Hepatitis C virus (HCV)
• F-protein of Respiratory syncytial virus (RSV)
• G-protein of Respiratory syncytial virus (RSV)

14. The use of any of claims 9-13, wherein the disease or disorder is an infectious, non-infectious, neoplastic, immune or metabolic disease or disorder.

15. The use of claim 14, wherein the infectious disease or disorder is a viral, bacterial, fungal, parasitic or prionic disease or disorder.

16. The use of any of claims 9-13, wherein the neoplastic disease or disorder is a cancer.

17. The use of claim 16, wherein the cancer is a sarcoma, a leukemia, a lymphoma, a myeloma, a melanoma, an adenoma, a carcinoma, a choriocarcinoma, a gastrinoma, a pheochromocytoma, a prolactinoma, adult T-cell leukemia/lymphoma or a neuroma.

18. The use of any of claims 9-17, wherein the immune disease or disorder is a disease or disorder **characterized by** immunosuppression.

19. The use of any of claims 9-18, wherein the animal is a mammal.

20. The use of claim 19, wherein the mammal is selected from a human, a chimpanzee, a cynomologous monkey, a gibbon, a simian monkey, a macaque monkey, a mouse, a rat, a cat, a dog, a horse, a rabbit, a camel, a llama, a ruminant, a horse, a pig.

21. The use of claim 20, wherein the ruminant is chosen from a cow, a bull, a goat, a sheep, a bison, a buffalo, a deer and a stag.

22. The use of any of claims 9-21, wherein the medicament is suitable for administration intraveneously, intramuscularly, intradermally, subcutaneously, intraperitoneally, parenterally, topically, locally, by gargling, by a patch, as a spray, sublingually, orally, by suppository, or by drops.

23. The use of any of claims 9-22, wherein the lipid vesicle is a virosome.

24. The use of claim 23, wherein the virosome is an immunopotentiating reconstituted influenza virosome ("influenza virosome"), or a chimeric virosome.

25. The use of claim 24, wherein the influenza virosome or the chimeric virosome is a chimeric influenza virosome.

26. A method of non-specifically stimulating the immune response of an animal to a disease or disorder, comprising administering a lipid vesicle according to any of claims 1-8.
